# EUROPÄISCHE PATENTANMELDUNG

(11) **EP 2 180 417 A1**
(43) Veröffentlichungstag der Anmeldung: **28.04.2010**
(21) Anmeldenummer: 08018500.2
(22) Anmeldetag: 22.10.2008
(51) Int. Cl.: G06F 19/00

(54) **Verfahren und System zur Therapie-Adhärenz-Überwachung**

(71) Anmelder: InterComponentWare AG, 69190 Walldorf (DE)
(72) Erfinder: Liedtke, Thomas, Dr., 69190 Walldorf (DE); Karbach, Philipp, 69190 Walldorf (DE)
(74) Vertreter: Müller-Boré & Partner Patentanwälte

(57) **Zusammenfassung**

Erfindung betrifft die Kontrolle bzw. Überwachung einer Verabreichung von Medikamenten für zumindest einen Patienten, wobei ein erfindungsgemäßes Verfahren umfasst:
- Bereitstellen eines medizinischen Verordnungsdatensatzes für den zumindest einen Patienten in einer Verordnungsdatenbank derart, dass der medizinische Verordnungsdatensatz für eine Vielzahl von Verordnungszeitpunkten jeweils einen Verabreichungsdatensatz festlegt, welcher einer Verabreichungsidentifikation Verordnungszeitdaten und zumindest eine Arzneimittelidentifikation zuordnet;
- Erfassen eines an einer Verabreichungseinheit angeordneten Verabreichungsidentifikationscodes, in welchem eine Verabreichungsidentifikation kodiert ist, mittels einer Verabreichungskontrolleinheit;
- Übermitteln von Verabreichungskontrolldaten von der Verabreichungskontrolleinheit an die Verordnungsdatenbank derart, dass die übermittelten Verabreichungskontrolldaten die erfasste Verabreichungsidentifikation und Verabreichungszeitdaten umfassen, welche den Zeitpunkt des Erfassens des Verabreichungsidentifikationscodes festlegen;
- Ermitteln desjenigen Verabreichungsdatensatz in der Verordnungsdatenbank, dessen in der Verordnungsdatenbank festgelegte Verabreichungsidentifikation mit der erfassten Verabreichungsidentifikation übereinstimmt; und
- Zuordnen der Verabreichungskontrolldaten zu dem ermittelten Verabreichungsdatensatz.

## Beschreibung

Die vorliegende Erfindung betrifft ein computer-implementiertes Verfahren, ein Computersystem und ein Computerprogrammprodukt zur automatischen Überprüfung bzw. Kontrolle bzw. Steuerung von Arzneimittelverordnungen für einen Patienten, insbesondere zur Überwachung zur automatischen Überprüfung bzw. Kontrolle bzw. Steuerung einer Therapie-Adhärenz.

Ältere Menschen insbesondere Bewohner eines Altenheimes erhalten oft eine Vielzahl von Medikamenten, die sie zum Teil in unterschiedlichen Verordnungen von wechselnden Ärzten verschrieben bekommen. Für die Bewohner gestaltet sich die Zuordnung der richtigen Medikamente zum jeweiligen Einnahmezeitpunkt als problematisch. Aufgrund fortgeschrittener Krankheiten oder altersbedingter Demenz ist ihnen oft nicht mehr zuverlässig möglich, die korrekte Stellung und Einnahme von Medikamenten einzuhalten. Daher wird das Stellen der Medikamente in Pflegeeinrichtungen von qualifizierten Pflegekräften übernommen. Gestellt bzw. in der richtigen Dosierung zur Einnahme vorbereitet werden Medikamente oft im Voraus für sieben Wochentage und vier Einnahmezeitpunkte (z.B. morgens, mittags, abends und nachts) je Wochentag (7x4-Schema). Für dieses 7x4-Schema gibt es auch vorgeformte Hilfen, sogenannten Dosetten, mit entsprechender Beschriftung. Für jeden Einnahmezeitpunkt ist ein Fach oder ein Döschen vorgesehen, in das die zu diesem Einnahmezeitpunkt einzunehmenden Medikamente von Hand einsortiert werden. Es gibt allerdings auch eine nicht unerhebliche Zahl von Patienten, für die aufgrund ihrer schweren Erkrankung das 7x4-Schema nicht anwendbar bzw. nicht ausreichend ist.

Für das korrekte Stellen der Medikamente werden genaue Angaben über den Namen des Medikaments oder eine sonstige eindeutige Identifikation des Medikaments, die Höhe der Dosierung und den Einnahmezeitpunkt benötigt. Diese Daten ergeben sich in der Regel aus der Verordnung durch einen Arztes und bilden insbesondere einen medizinischen Verordnungsplan. Dieser wird vorzugsweise nach einem geläufigen Schema angegeben, z.B. steht "Aspirin 1-1-1-0" für Aspirin jeweils morgens ("1" an der ersten Stelle der Zahlenreihe), mittags ("1" an der zweiten Stelle der Zahlenreihe) und abends ("1" an der dritten Stelle der Zahlenreihe) eine Tablette. Nachts ist keine Tablette verordnet ("0" an der vierten Stelle der Zahlenreihe). Die folgende Tabelle zeigt eine beispielhaften Wochenplan:

| Medikament | Mo | Di | Mi | Do | Fr | Sa | So |
|---|---|---|---|---|---|---|---|
| Delix® 5 plus | 1-0-½ -0 | 1-0-½ -0 | 1-0-½ -0 | 1-0-½ -0 | 1-0-½ -0 | 1-0-½ -0 | 1-0-½ -0 |
| Digimerck® minor 0,07mg Tbl. | 0-1-0-0 | | 0-1-0-0 | | 0-1-0-0 | | |
| isoket® retard 120mg Retardkaps. | 1-0-0-0 | 1-0-0-0 | 1-0-0-0 | 1-0-0-0 | 1-0-0-0 | 1-0-0-0 | 1-0-0-0 |

Dies hieße übersetzt in einem 7x4-Schema:

| | Mo | Di | Mi | Do | Fr | Sa | So |
|---|---|---|---|---|---|---|---|
| morgens | 1 Delix | 1 Delix | 1 Delix | 1 Delix | 1 Delix | 1 Delix | 1 Delix |
| | 1 isoket | 1 isoket | 1 isoket | 1 isoket | 1 isoket | 1 isoket | 1 isoket |
| mittags | 1 Digimerck | | 1 Digimerck | | 1 Digimerck | | |
| abends | ½ Delix | ½ Delix | ½ Delix | ½ Delix | ½ Delix | ½ Delix | ½ Delix |
| nachts | | | | | | | |

Im Durchschnitt erhalten Pflegeheimbewohner 6-7 Dauermedikamente und zusätzlich noch eine Akutmedikation. Das bedeutet, dass in Pflegeheimen für durchschnittlich 60-70 Patienten 6-7 Medikamente gestellt werden müssen. Dies geschieht in der Regel in der Spät- oder Nachtschicht, da hier der Aufwand für die Pflege geringer ist. Aufgrund der hohen Komplexität des Stellens ist der Prozess sehr zeitaufwändig und mitunter fehleranfällig. Einige Pflegeheime sind dazu übergegangen, das Stellen der Medikamente an die Apotheke abzugeben. Die Versorgung der Heime mit gestellten Arzneimitteln beispielsweise für eine Woche durch die Apotheke führt letztendlich zu einer Verlagerung des manuellen Stellens von Medikamenten in die Apotheke. Zunehmend gehen Apotheken dazu über, ihre Prozesse beim Stellen der Medikamente zu verbessern, z.B. indem Sie statt Dosetten im 7x4-Schema die Wochenration der Medikamente manuell verblistern, z.B. in sogenannten Kartenblistern.

Andere wiederum sahen die Notwendigkeit für eine Automatisierung der Prozesse mit speziell dafür entworfenen Sortiermaschinen, einer sogenannten Blistermaschine. Diese Blistermaschinen werden autark in den Apotheken aufgestellt und mit Medikamenten gefüllt. Dies geschieht mit kleinen Kassetten für jeweils ein Medikament. Die Kassetten verfügen über eine speziellen Dispenser, der die Medikamente auf Befehl herausfallen lässt. Im unteren Bereich der Maschine werden die herausgefallenen Medikamente in einem Tütchen aufgefangen und gemeinsam verschweißt. Durch die manuelle Eingabe der Verordnungspläne der Patienten weiß die Maschine welche Medikamente zu welchem Zeitpunkt herausfallen müssen, um gemeinsam verschweißt zu werden. Die einzelnen Tütchen werden vor dem Verschweißen von einem Drucker bedruckt.

Bei der individuellen Verblisterung von Arzneimitteln für einen Patienten werden verschiedene Medikamente unterschiedlicher Hersteller gemeinsam pro Dosierungszeitpunkt verpackt. Außerdem werden die Medikamente für einen Patienten oft von unterschiedlichen Ärzten verschrieben. Schließlich ist die Kapazität der bei der automatisierten Verblisterung eingesetzten Maschinen je nach Maschine beispielsweise auf einige Hundert verschiedene Medikamente begrenzt. All dies führt sehr schnell zu einer Grenze der Verlässlichkeit, Sicherheit und Flexibilität bei der individuellen Versorgung von Patienten mit Medikamenten. Vor allem eine sichere Zuordnung von Medikamenten und eine Überwachung, Dokumentation und Kontrolle für die Verabreichung der individuell verblisterten Medikamente ist sehr zeitaufwändig und aufgrund der vereinheitlichten Verpackung in Form von Blistertütchen fehlerträchtig.

Es ist Aufgabe der vorliegenden Erfindung, ein Verfahren, ein Computersystem und ein Computerprogrammprodukt bereitzustellen, die eine schnellere, zuverlässigere und insbesondere für die Benutzung durch mehrere Benutzer besonders flexible und überschaubare Versorgung von Patienten mit Medikamenten gewährleisten.

Diese Aufgabe wird durch computer-implementierte Verfahren zur Kontrolle und/oder Überwachung einer Verabreichung von Medikamenten bzw. zur individuellen Verblisterung mit den in den Ansprüchen 1 bzw. 10 angegebenen Merkmalen, einen Dispenser nach Anspruch 11 sowie ein Computersystem und ein Computerprogrammprodukt mit den in den Ansprüchen 13 bzw. 15 angegebenen Merkmalen gelöst. Bevorzugte Ausführungsformen sind Gegenstand der abhängigen Unteransprüche.

Somit stellt die Erfindung ein computer-implementiertes Verfahren zur Kontrolle bzw. Überwachung einer Verabreichung von Medikamenten an und/oder für zumindest einen Patienten bereit, welches ein Bereitstellen eines medizinischen Verordnungsdatensatzes für den zumindest einen Patienten in einer insbesondere zentralen Verordnungsdatenbank derart umfasst, dass der medizinische Verordnungsdatensatz für eine Vielzahl von Verordnungszeitpunkten jeweils einen Verabreichungsdatensatz festlegt, welcher einer Verabreichungsidentifikation Verordnungszeitdaten und zumindest eine Arzneimittelidentifikation zuordnet. Die Verabreichungsidentifikation ist dabei derart eindeutig bzw. einzigartig festgelegt, dass sie eine eindeutige Identifikation des jeweiligen Verabreichungsdatensatzes innerhalb der Verordnungsdatenbank erlaubt. Vorzugsweise ist die Verabreichungsidentifikation als eindeutige Verabreichungsidentifikationsnummer festgelegt.

Außerdem umfasst das Verfahren ein Erfassen eines an einer Verabreichungseinheit angeordneten Verabreichungsidentifikationscodes, in welchem eine zu erfassende Verabreichungsidentifikation kodiert ist, mittels einer Verabreichungskontrolleinheit. Die Verabreichungskontrolleinheit wird dabei insbesondere an einem dezentralen, patientennahen Platz, wie z.B. beim Patienten vor Ort (z.B. zu Hause) und/oder im Pflegeheim und/oder einem Krankenhaus und/oder in der Arztpraxis vorgesehen. Die Verabreichungskontrolleinheit kann dabei fest installiert oder mobil sein. Insbesondere könnte beispielsweise ein mobiler Pflegedienst eine mobile Verabreichungskontrolleinheit zum Patienten mitnehmen.

Als Verabreichungseinheit wird insbesondere ein Behältnis zur Aufnahme von Medikamenten für die Verabreichung an den Patienten zu einem jeweiligen Verabreichungszeitpunkt bereitgestellt. Dies könnte beispielsweise ein Döschen oder Schächelchen oder ein Fach in einer Dosette sein. Besonders bevorzugt wird als Verabreichungseinheit eine individuell für einen Patienten gefüllte Blistertüte bereitgestellt, auf der bzw. an der der Verabreichungsidentifikationscode angeordnet, insbesondere aufgedruckt ist.

Außerdem umfasst das Verfahren ein Übermitteln von Verabreichungskontrolldaten von der Verabreichungskontrolleinheit an die Verordnungsdatenbank derart, dass die übermittelten Verabreichungskontrolldaten die erfasste Verabreichungsidentifikation und Verabreichungszeitdaten umfassen, wobei die Verabreichungszeitdaten den Zeitpunkt des Erfassens des Verabreichungsidentifikationscodes insbesondere nach Art eines Zeitstempels festlegen. Außerdem umfasst das Verfahren ein Ermitteln desjenigen Verabreichungsdatensatzes in der Verordnungsdatenbank, dessen in der Verordnungsdatenbank festgelegte Verabreichungsidentifikation mit der erfassten Verabreichungsidentifikation übereinstimmt und ein insbesondere automatisches Zuordnen der Verabreichungskontrolldaten zu dem ermittelten Verabreichungsdatensatz. Vorzugsweise werden die Verabreichungskontrolldaten zusammen mit dem Verabreichungsdatensatz gespeichert. Dies muss aber nicht unmittelbar erfolgen, sondern kann in einer bevorzugten Ausführungsform von einer Eigabe einer Verifikationsbestätigung durch einen Benutzer abhängig gemacht werden.

Der Verordnungsdatensatz identifiziert dabei insbesondere einen medizinischen Verordnungsplan für den zumindest einen Patienten, d.h. er legt für eine Vielzahl von regelmäßigen oder unregelmäßigen Verabreichungszeitpunkten die für eine verordnete Therapie zu verabreichenden Medikamente und ihre Dosierung fest. In der Medizin stellen die mangelnden Überwachungsmöglichkeiten der Therapie-Adhärenz ein großes Problem dar. Letztendlich kann sich der für die Behandlung Verantwortliche nur mit begrenzten Mitteln darüber informieren, ob der Patient die verordneten Maßnahmen unternommen hat oder nicht. Im häufigsten Fall wird dies die Verordnung von Medikamenten und deren korrekte Einnahme nach dem verordneten Einnahmemuster betreffen. Insbesondere im Fall einer patientenindividuellen Verblisterung von Arzneimitteln, wobei industriell mehrere Medikamente gemeinsam pro Dosierungszeitpunkt verpackt werden, würde eine unterlassene Verabreichung bzw. Einnahme oder eine falsche Zuordnung zu einem Patienten oft gleich mehrere Medikamente betreffen und somit tendenziell sogar eine größere Gefahr darstellen als bei einzelnen Medikamentendosierungen. Die tatsächliche Einnahme der Medikamente lässt sich durch technische Lösungen nur schwer vollständig nachvollziehen. Die Erfindung bietet aber technische Hilfsmittel insbesondere im Patientenumfeld, die eine schnellere und insbesondere für die Benutzung durch mehrere Patienten und/oder Pflegepersonen eine höhere Zuverlässigkeit bei der Versorgung von Patienten mit Medikamenten gewährleisten.

Vorzugsweise wird der Verabreichungsidentifikationscode als optisch lesbarer Code an der Verabreichungseinheit bereitgestellt bzw. angeordnet. Das Erfassen des Verabreichungsidentifikationscodes erfolgt dabei vorzugsweise mittels eines optischen Lesegeräts der Verabreichungskontrolleinheit. Als optisch lesbarer Code wird vorzugsweise ein 1 D-Code, insbesondere ein Strichcode, und/oder ein 2D-Code, insbesondere ein Array-Code oder gestapelter Code oder Matrixcode, an der Verabreichungseinheit angeordnet. In einer anderen bevorzugten Ausführungsform werden die Verabreichungsidentifikationsdaten bzw. der Verabreichungsidentifikationscode in einem RFID-chip an der Verabreichungseinheit angeordnet. Zum Erfassen des Verabreichungsidentifikationscodes umfasst die Verabreichungskontrolleinheit dabei vorzugsweise einen RFID-Empfänger.

Vorzugsweise wird in der Verordnungsdatenbank für eine Vielzahl von Patienten eine Vielzahl von Verordnungsdatensätzen (insbesondere jeweils ein Verordnungsdatensatz pro Patient) bereitgestellt, denen in der Verordnungsdatenbank jeweils eine Patientenidentifikation zugeordnet ist, wobei das Verfahren außerdem umfasst:
- Bereitstellen einer Patientendatenbank, welche für eine Vielzahl von Patienten jeweils einer Patientenidentifikation Patientenverifikationsdaten (z.B. Patientenname, Patientenfoto) zuweist bzw. zuordnet;
- Ermitteln derjenigen Patientenverifikationsdaten in der Patientendatenbank, deren Patientenidentifikation in der Verordnungsdatenbank demjenigen Verordnungsdatensatz zugeordnet ist, der den ermittelten Verabreichungsdatensatz umfasst;
- Übermitteln der ermittelten Patientenverifikationsdaten an die Verabreichungskontrolleinheit; und
- Ausgeben der übermittelten Patientenverifikationsdaten mittels einer Ausgabeeinrichtung der Verabreichungskontrolleinheit.

In einer weiteren bevorzugten Ausführungsform umfasst das Verfahren außerdem:
- Bereitstellen einer Arzneimitteldatenbank, welche für eine Vielzahl von Arzneimitteln jeweils einer Arzneimittelidentifikation Arzneimittelverifikationsdaten (z.B. Arneimittelbezeichnung, Arzneimittelfoto oder schematische Zeichnung bzw. graphische Darstellung der geometrischen Formen und Besonderheiten des jeweiligen Medikaments (Form, Größe, Farbe, Prägungen, etc.)) zuweist bzw. zuordnet;
- Ermitteln eines Arzneimittelverifikationsdatensatzes derart, dass der Arzneimittelverifikationsdatensatz einige, vorzugsweise alle Arzneimittelverifikationsdaten umfasst, die den im ermittelten Verabreichungsdatensatz der erfassten Verabreichungsidentifikation zugeordneten

Arzneimittelidentifikationen zugeordnet sind;
- Übermitteln des ermittelten Arzneimittelverifikationsdatensatzes an die Verabreichungskontrolleinheit; und
- Ausgeben der vom übermittelten Arzneimittelverifikationsdatensatz umfassten Arzneimittelverifikationsdaten mittels einer Ausgabeeinrichtung der Verabreichungskontrolleinheit.

Im allgemeinen umfasst das Verfahren damit vorzugsweise:
- Ermitteln von Verabreichungsverifikationsdaten aus den übermittelten Verabreichungskontrolldaten;
- Übermitteln des ermittelten Verabreichungsverifikationsdaten an die Verabreichungskontrolleinheit; und
- Ausgeben der übermittelten Verabreichungsverifikationsdaten zumindest teilweise mittels einer Ausgabeeinrichtung der Verabreichungskontrolleinheit.

Dabei umfassen die Verabreichungsverifikationsdaten vorzugsweise Patientenverifikationsdaten und/oder Arzneimittelverifikationsdaten.

Vorzugsweise umfasst das Verfahren außerdem:
- Erfassen der Eingabe einer Verifikationsbestätigung an der Verabreichungskontrolleinheit;
- Übermitteln der Verifikationsbestätigung an die Verordnungsdatenbank; und
- Speichern der zugeordneten Verabreichungskontrolldaten zusammen mit dem ermittelten Verabreichungsdatensatz in der Verordnungsdatenbank.

In einer bevorzugten Ausführungsform umfasst das Bereitstellen eines medizinischen Verordnungsdatensatzes für den zumindest einen Patienten ein Festlegen eines Dringlichkeitsparameters für den zumindest einen Patienten und/oder für zumindest einen Verabreichungsdatensatz des zumindest einen Patienten, wobei das Verfahren außerdem umfasst:
- insbesondere ständiges oder regelmäßiges Überwachen der Verordnungszeitdaten; und/oder
- Ausgeben einer Warnmeldung, falls nicht innerhalb eines vom Dringlichkeitsparameter abhängigen Toleranzzeitintervalls nach einem in den Verordnungszeitdaten festgelegten Verordnungszeitpunkt dem Verabreichungsdatensatz Verabreichungskontrolldaten zugeordnet wurden.

Vorzugsweise erfolgt das Erfassen des Verabreichungsidentifikationscodes mittels eines Mobiltelefons mit integrierter Kamera und das Übermitteln der Verabreichungskontrolldaten über ein Mobilfunknetz. In diesem Aspekt betrifft die Erfindung somit die Verwendung eines Mobiltelefons mit integrierter Kamera zur als Verabreichungskontrolleinheit in einem Verfahren gemäß der vorliegenden Erfindung oder eine bevorzugten Ausführungsform davon.

Vorzugsweise werden die Verabreichungskontrolldaten für eine Vielzahl von Verabreichungseinheiten in der Verabreichungskontrolleinheit zumindest temporär gespeichert bzw. zwischengespeichert bevor sie an die Verordnungsdatenbank übermittelt werden. Dabei werden vorzugsweise Verabreichungskontrolleinheit für eine Vielzahl von Verabreichungseinheiten gespeichert bevor sie vorzugsweise im wesentlichen gemeinsam übermittelt werden.

Vorzugsweise wird die Verabreichungskontrolleinheit als zumindest ein Teil eines Dispensers für eine Vielzahl von Verabreichungseinheiten derart bereitgestellt, dass das Erfassen des an einer Verabreichungseinheit angeordneten Verabreichungsidentifikationscodes beim Ausgeben bzw. Entnehmen der Verabreichungseinheit vom bzw. aus dem Dispenser automatisch erfolgt. Insbesondere wird die vorzugsweise eine Blisterbox als Verabreichungskontrolleinheit bereitgestellt.

Außerdem betrifft die Erfindung ein computerimplementiertes Verfahren zur individuellen Verblisterung von Medikamenten für zumindest einen Patienten, welches umfasst:
- ein Verfahren zur Erstellung und/oder Optimierung eines medizinischen Verordnungsplans;
- Ermitteln zumindest eines Verabreichungsdatensatzes aus dem medizinischen Verordnungsplan;
- automatisches Generieren einer Verabreichungsidentifikation für den zumindest einen Verabreichungsdatensatz;
- Speichern des Verabreichungsdatensatz zusammen mit der Verabreichungsidentifikation in einer zentralen Verordnungsdatenbank;
- Übermitteln des Verabreichungsdatensatzes zusammen mit der jeweiligen Verabreichungsidentifikation und/oder eines daraus generierten Verabreichungsidentifikationcodes an eine automatisch gesteuerte Verblisterungsanlage;
- automatisches Verblistern von Medikamenten gemäß dem Verabreichungsdatensatz, wobei das automatische Verblistern ein Anordnen eines/des aus der Verabreichungsidentifikation generierten Verabreichungsidentifikationcodes an den Blister umfasst.

Außerdem bietet die Erfindung einen Dispenser, insbesondere eine Blisterbox, zum Bereitstellen von individuell verblisterten Medikamenten, umfassend:
- einen Aufnahmebehälter zum Aufnehmen einer Vielzahl von Verabreichungseinheiten, insbesondere Blistertütchen, insbesondere in Form von Schlauchblistern;
- eine Auszugschacht zum Entnehmen einzelner Verabreichungseinheiten, insbesondere einzelner Blistertütchen;
- eine Leseeinrichtung zum Auslesen von an den Verabreichungseinheiten angeordneten Verabreichungsidentifikationcodes; und
- eine integrierte Zeitmesseinrichtung zum Erfassen von Verabreichungszeitdaten, welche den Zeitpunkt des Erfassens eines Verabreichungsidentifikationcodes zumindest teilweise bzw. relativ (z.B. in Form von Zeitintervallen) festlegen.

Vorzugsweise ist der Aufnahmebehälter als Plastikbehälter bzw. Plastikbox mit ausreichend Platz für eine Wochendosis aufgerollter Schlauchblister ausgebildet. Außerdem umfasst der Dispenser vorzugsweise einen Deckel. In den Auszugschacht, der vorzugsweise im Deckel ausgebildet ist, lässt sich vorzugsweise ein Schlauchblister einfädeln. Die Leseeinrichtung ist vorzugsweise als 1 D/2D-Barcode-Scanner und/oder als RFID-Leser ausgebildet und insbesondere im bzw. am Deckel angeordnet. Vorzugsweise weist der Dispenser eine Führung für die zu entnehmenden Blistertütchen derart auf, dass diese beim Entnehmen durch den Auszugschacht so an der Leseeinrichtung vorbei geführt werden, dass diese den Verabreichungsidentifikationscode lesen kann. Vorzugsweise weist der Dispenser eine Speichereinrichtung (z.B. SD, Flash) zum Speichern von Verabreichungskontrolldaten vorzugsweise für ein Wochenration verblisterter Medikamente. Alternativ oder zusätzlich umfasst der Dispenser vorzugsweise eine Datenübertragungsschnittstelle (z.B. ZigBee, WiFi, UMTS/GPRS, USB, ...) um Verabreichungskontrolldaten zu übertragen. Dies könnten je nach Anwendung die unmittelbar erfassten Verabreichungskontrolldaten (für eine Echtzeit- bzw. online-Anwendung) und/oder die gespeicherten Verabreichungskontrolldaten (bei einer nicht dauernd vernetzten Anwendung) sein. Vorzugsweise umfasst der Dispenser außerdem eine Ausgabeeinrichtung zum Ausgeben von Verabreichungsverifikationsdaten und/oder eine Eingabeeinrichtung zum Eingeben einer Verifikationsbestätigung.

In einem weiteren Aspekt betrifft die Erfindung ein Computersystem zur Kontrolle bzw. Überwachung einer Verabreichung von Medikamenten für zumindest einen Patienten, umfassend:
- eine insbesondere zentrale Verordnungsdatenbank zum Bereitstellen eines medizinischen Verordnungsdatensatzes für den zumindest einen Patienten derart, dass der medizinische Verordnungsdatensatz für eine Vielzahl von Verordnungszeitpunkten jeweils einen Verabreichungsdatensatz festlegt, welcher einer eindeutig festgelegten Verabreichungsidentifikation, insbesondere einer Verordnungsidentifikationsnummer bzw. Verabreichungsidentifikationsnummer, Verordnungszeitdaten und zumindest eine Arzneimittelidentifikation zuordnet;
- eine Verabreichungskontrolleinheit, welche insbesondere an einem dezentralen, patientennahen Platz eingesetzt wird bzw. einsetzbar ist, zum Erfassen eines an einer Verabreichungseinheit angeordneten Verabreichungsindentifikationscodes, in welchem eine zu erfassende Verabreichungsidentifikation kodiert ist, wobei die Verabreichungskontrolleinheit eine Systemuhr umfasst und ausgelegt ist den Zeitpunkt eines Erfassens des Verabreichungsidentifikationscodes als Verabreichungszeitdaten, insbesondere nach Art eines Zeitstempels, zu erfassen;
- ein Datenübertragungsnetz zum Übermitteln von Verabreichungskontrolldaten von der Verabreichungskontrolleinheit an die Verordnungsdatenbank derart, dass die übermittelten Verabreichungskontrolldaten die erfasste Verabreichungsidentifikation und Verabreichungszeitdaten umfassen;
- eine Analyseeinrichtung zum Ermitteln desjenigen Verabreichungsdatensatz in der Verordnungsdatenbank, dessen in der Verordnungsdatenbank festgelegte Verabreichungsidentifikation mit der erfassten Verabreichungsidentifikation übereinstimmt; und
- eine Zuordnungseinheit zum insbesondere automatischen Zuordnen der Verabreichungskontrolldaten zu dem ermittelten Verabreichungsdatensatz.

Vorzugsweise ist das Computersystem ausgelegt, ein Verfahren gemäß der vorliegenden Erfindung in einer oder mehreren der beschriebenen Ausführungsformen auszuführen.

Die Erfindung bietet außerdem ein Computerprogrammprodukt, insbesondere in Form eines maschinenlesbaren Mediums, als Signal und/oder als Datenstrom ausgestaltet, welches maschinenlesbaren Programmcode umfasst, der, wenn er geladen wird auf einem Computer, zur Ausführung eines Verfahrens gemäß der vorliegenden Erfindung in einer oder mehreren der beschriebenen Ausführungsformen führt bzw. geeignet ist.

Die Erfindung wird nachfolgend anhand bevorzugter Ausführungsformen mit Bezug auf die begleitende Zeichnungen beschrieben. Dabei zeigen:
- Fig.1: eine schematische Darstellung einer bevorzugen Ausführungsform eines erfindungsgemäßen Systems;
- Fig. 2: eine schematische Darstellung eines Verfahrens gemäß einer bevorzugten Ausführungsform der vorliegenden Erfindung;
- Fig.3A-C: zeigen beispielhafte Ausschnitte von Bildschirmdarstellungen zur Veranschaulichung der Erfindung anhand bevorzugter Ausführungsformen;
- Fig. 4: eine schematische Darstellung eines beispielhaften Systems zum Implementieren der Erfindung.

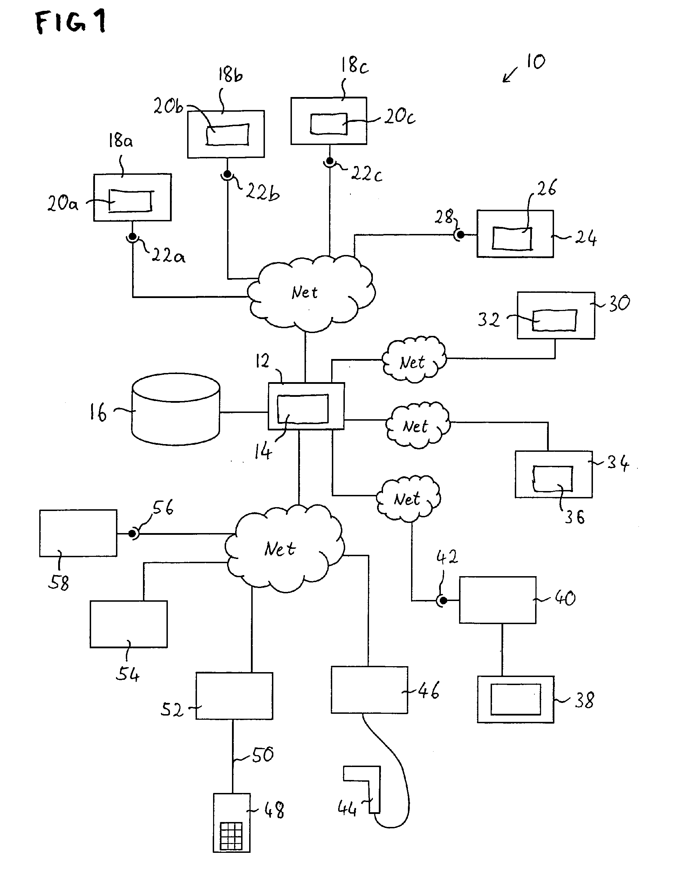

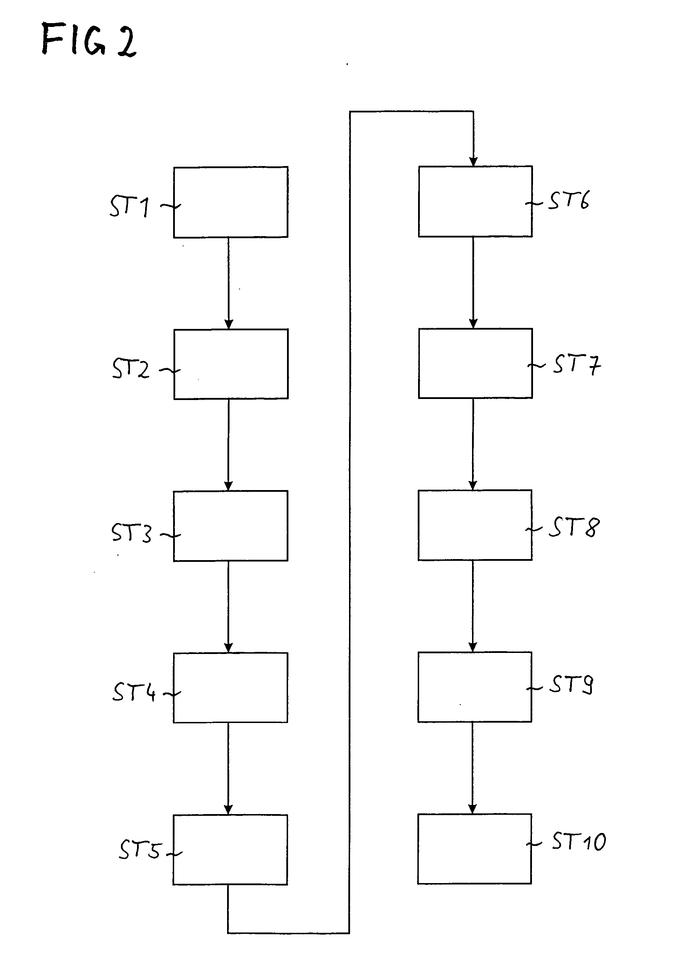

**Fig. 1** zeigt eine schematische Darstellung eines Systems 10 gemäß einer bevorzugten Ausführungsform der Erfindung. Anhand dieser Darstellung lässt sich auch das erfindungsgemäße Verfahren verdeutlichen, das in einer bevorzugten Ausführungsform parallel dazu mit Bezug auf **Fig. 2** beschrieben wird. Gemäß der in Fig. 1 gezeigten bevorzugten Ausführungsform umfasst das System 10 einen zentralen Applikationsserver 12, auf dem eine zentrale Anwendung 14 abläuft bzw. gehostet ist. Der zentrale Applikationsserver 12 kann dabei wiederum aus mehreren Komponenten bestehen und die zentrale Anwendung 14 kann mehrere Module umfassen. In der gezeigten Ausführungsform steht der zentrale Applikationsserver 12 mit einem zentralen Datenspeicher 16 in Verbindung, auf welchem insbesondere Daten einer Verordungsdatenbank und/oder einer Patientendatenbank und/oder einer Arzneimitteldatenbank gespeichert sind. Die Verordungsdatenbank und/oder die Patientendatenbank und/oder die Arzneimitteldatenbank können in einer zentralen Datenbank gemeinsam implementiert sein und einzeln oder als zentrale Datenbank gemeinsam vom zentralen Applikationsserver gelesen und/oder beschrieben, insbesondere gesteuert oder verwaltet werden. Der zentrale Datenspeicher kann dabei eine oder mehrere logische Einheiten bilden und insbesondere mehrere verteilte Speicherkomponenten umfassen. Der zentrale Applikationsserver 12 ist in ein Netzwerk eingebunden und steht insbesondere über das Internet (in Fig. 1 als "Net" bezeichnet) mit einer Vielzahl dezentraler Systeme in Verbindung, wobei die Kommunikation vorzugsweise verschlüsselt (z.B. VPN) erfolgt. Alternativ oder zusätzlich können auch vom Internet unabhängige, gesicherte Netze bereitgestellt werden.

In der dargestellten, bevorzugten Ausführungsform ist eine Vielzahl dezentraler Anwendersysteme 18a, 18b, 18c vorgesehen, auf denen jeweils eigenständige Anwendungen 20a, 20b, 20c laufen. Über diese Anwendersysteme 18a, 18b, 18c werden in einem ersten Schritt ST1 insbesondere Stammdaten bzw. Patientendaten für eine Vielzahl von Patientendaten erfasst bzw. gepflegt und in einer Gesundheitsdatenbank, insbesondere der Patientendatenbank, hinterlegt. Diese Patientendaten bzw. Patientenstammdaten umfassen vorzugsweise eine eindeutige Patientenidentifikation, z.B. in Form einer eindeutigen Identifikationsnummer. Außerdem umfassen die Patientendaten vorzugsweise Versicherungsdaten der Patienten und/oder Patientenkontrolldaten. Vorzugsweise umfassen die Patientenkontrolldaten ein Foto bzw. Passbild und/oder den Namen und/oder das Geburtsdatum des jeweiligen Patienten. Vorzugsweise sind die Patientenkontrolldaten zumindest teilweise als Bilddaten bzw. Grafikdaten bzw. Videodaten und/oder als Audiodaten von den Patientendaten umfasst. Die dezentralen Anwendersysteme können dabei beispielsweise einem Krankenversicherer, einem Pflegedienst und/oder dem bzw. den Patienten selbst zur Verfügung stehen. Für eine sichere und zuverlässige Datenübertragung bzw. Datenkonversion zwischen den eigenständigen Anwendungen 20a, 20b, 20c und dem zentralen Applikationsserver 12 bzw. der zentralen Anwendung 14 ist jeweils eine geeignete Schnittstelle 22a, 22b, 22c vorgesehen.

In einem weiteren Schritt ST2 wird/werden vorzugsweise eine Verordnung bzw. Verordnungsdaten für zumindest einen Patienten über ein erstes Verordnersystem 24 erfasst, wobei die Verordnungsdaten für zumindest ein Medikament eine Arzneimittelidentifikation und vorzugsweise -dosierung und/oder eine Verabreichungsform umfassen. Besonders bevorzugt umfassen die Verordnungsdaten Verordnungszeitdaten für das zumindest eine Medikament, welche festlegen, zu welcher Zeit der jeweilige Patient dieses Medikament anwenden soll. Hierzu ist auf dem Verordnersystem 24 vorzugsweise eine eigenständige Verordneranwendung 26 vorgesehen, über welche die Verordnungsdaten eingegeben werden können und welche die Verordnungsdaten über eine Schnittstelle 28 zur zentralen Anwendung 14 übertragen kann. Vorzugsweise steht das erste Verordnersystem 24 einem Arzt zur Verfügung. Alternativ oder zusätzlich kann für denselben oder einen anderen Arzt ein zweites Verordnersystem 30 vorgesehen sein, auf dem ein Browser 32 läuft, über den die vorzugsweise Web-basierte zentrale Anwendung 14 im Umfang der für das zweite Verordnersystem 30 festgelegten Freigabespezifikationen bzw. Autorisierungen bedient bzw. gesteuert bzw. ausgeführt werden kann. Vorzugsweise lassen sich über die im Browser 32 verfügbare Benutzeroberfläche die im Zusammenhang mit dem ersten Verordnersystem 24 beschriebenen Eingaben vornehmen.

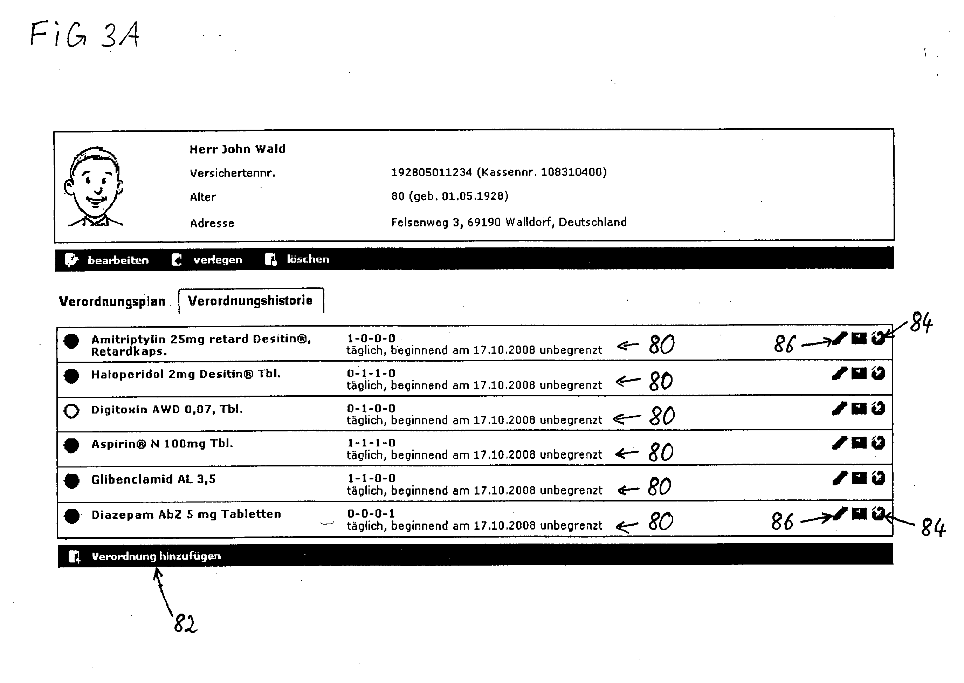

Zur Veranschaulichung zeigt **Fig. 3A** eine graphische Benutzeroberfläche einer Eingabeschnittstelle und/oder Ausgabeeinheit gemäß einer bevorzugte Ausführungsform eines Verordnersystems 30. Dabei wird dem Benutzer, z.B. dem Arzt ein medizinischer Verordnungsplan einer Patientin mit einer Vielzahl von Verordnungen 80 in einer Liste, z.B. in einzelnen Reihen, präsentiert. Durch Betätigen (z.B. per Mausklick) eines Eingabefeldes 82 kann der Benutzer eine weitere Verordnung hinzufügen, durch Betätigen entsprechender "Löschen"-Felder 84 wieder aus dem Verordnungsplan entfernen. Durch Betätigen von Editierfeldern 86 kann er eine vorbestimmte bzw. vorbestimmbare (bevorzugt jede) Verordnung einzeln oder zusammen mit einer oder mehreren weiteren vorbestimmten bzw. vorbestimmbaren Verordnungen editieren. Vorzugsweise umfassen die Verordnungsdaten 80 zumindest eine Arzneimittelidentifikation. Diese ist in Fig. 3A nicht dargestellt, sondern wird vorzugsweise erst beim Editieren einzelner Verordnungsdaten bzw. Verordnungen für den Benutzer sichtbar bzw. direkt zugänglich. Ansonsten werden die Arzneimittelidentifikation insbesondere als interne Parametern genutzt.

In einem weiteren Schritt ST3 werden die im Schritt ST2 erfassten Verordnungsdaten einem Verordnungsplan des entsprechenden Patienten hinzugefügt bzw. der Verordnungsplan wird entsprechen ergänzt oder korrigiert. Dieser Verordnungsplan wird schließlich in Form eines Verordnungsdatensatzes für den Patienten in der Verordnungsdatenbank gespeichert.

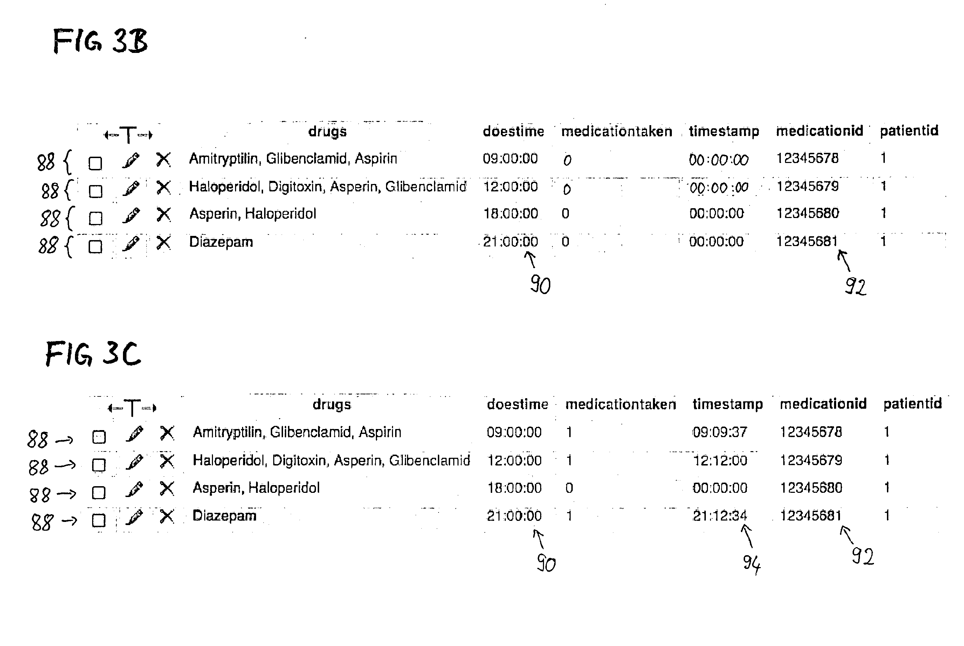

In einem weiteren Schritt ST4 werden vorzugsweise von einer Apotheke über ein Bestellsystem 34 Bestelldaten für die individuelle Verblisterung von Medikamenten für einzelne Patienten insbesondere auf Basis der in der Verordnungsdatenbank hinterlegten Verordnungsdatensätze erstellt. Dazu umfasst ein Verordnungsdatensatz für einen Patienten eine Vielzahl von Verabreichungsdatensätzen. **Fig. 3B** zeigt eine graphische Benutzeroberfläche wie sie unter anderem in einem Browser 36, welcher auf dem Bestellsystem 34 läuft, verfügbar sein könnte. Diese Benutzeroberfläche repräsentiert eine Darstellung zumindest eines Teils eines Verordnungsdatensatzes auf Basis des Verordnungsplans von Fig. 3A. Dieser Verordnungsdatensatz umfasst eine Vielzahl von Verabreichungsdatensätzen 88, von denen jeder als eine Zeile bzw. Reihe in Fig. 3B dargestellt ist. Jeder Verabreichungsdatensatz 88 umfasst zumindest eine Arzneimittelidentifikation für die jeweiligen in der Spalte "drugs" angegebenen Arzneimittel. Außerdem umfasst jeder Verabreichungsdatensatz Verordnungszeitdaten 90, welche festlegen, wann die im jeweiligen Verabreichungsdatensatz festgelegten Arzneimittel vom Patienten eingenommen werden sollen. Dabei wird insbesondere jedem Verabreichungsdatensatz genau eine Uhrzeit bzw. Tageszeit zugeordnet. Im System 10 erfolgt somit eine Umstrukturierung der Verordnungsdaten von dem in Fig.3A dargestellten Verordnungsplan mit einer Vielzahl von Verordnungen 80, welche jeweils ein Medikament für einen oder mehrere Verabreichungszeitpunkte festlegen, hin zum Verordnungsdatensatz von Fig.3B mit einer Vielzahl von Verabreichungsdatensätzen 88, welche jeweils einen Verabreichungszeitpunkt mit einem oder mehreren Medikamenten verknüpfen.

Wie in Fig. 3B dargestellt umfasst jeder Verabreichungsdatensatz 88 eine Verabreichugnsidentifikation 92, insbesondere in Form einer Verabreichungsidentifikationsnummer oder in einer anderen digitalisierbaren Form, die eine eindeutige Identifikation des Verabreichungsdatensatzes zulässt. Vorzugsweise wird die Verabreichungsidentifikation 92 vom System 10 automatisch generiert. In einer bevorzugten Ausführungsform wird die Verabreichungsidentifikation 92 im Schritt ST3 des Erstellens bzw. Erweiterns bzw. Aktualisierens des Verordnungsplans automatisch generiert bzw. aktualisiert. In einer anderen bevorzugten Ausführungsform wird die Verabreichungsidentifikation 92 im Schritt ST4 des Erstellens von Bestelldaten für die individuelle Verblisterung von Medikamenten für einzelne Patienten mittels des Bestellsystems 34 vom System 10 automatisch generiert.

Die in Schritt ST4 erstellten Bestelldaten dienen als Basis für Verblisterungsaufträge, welche mittels des Systems 10 in einem Schritt ST5 an Blisterzentren bzw. Blisterautomaten 38 übermittelt werden. Dabei wird ein Blisterautomat 38 durch das System 10 insbesondere mittels einer Steuerungssoftware 40 über eine Schnittstelle 42 derart gesteuert, dass für jeden Verabreichungsdatensatz 88, d.h. für jede Einnahme von Medikamenten durch den Patienten bzw. jeden Einnahmezeitpunkt ein Blisterpäckchen bzw. Blistertüte mit den im Verabreichungsdatensatz festgelegten Medikamenten, insbesondere in der festgelegten Dosierung befüllt wird. Damit erhält der Patient mit einem einzigen Tütchen vorzugsweise alle für eine Einnahme erforderlichen Medikamente, weshalb jedes Tütchen eine Verabreichungseinheit bildet. Außerdem wird mit dem Blisterauftrag für jeden Verabreichungsdatensatz die zugehörige Verabreichungsidentifikation an den Blisterautomaten 38 bzw. die Steuerungssoftware 34 übermittelt. Die Verabreichungsidentifikation wird dann in codierter Form als Verabreichungsidentifikationscode an das Blistertütchen angebracht, insbesondere zusammen mit weiteren patientenspezifischen Daten auf das Blistertütchen aufgedruckt. Dieser Verabreichungsidentifikationscode ermöglicht damit eine eindeutige Identifikation jedes Blistertütchens. Der Verabreichungsidentifikationscode umfasst vorzugsweise einen 1D- und/oder 2D-Strichcode bzw. einen Matrixcode. In einer anderen bevorzugten Ausführungsform wird an jedem Blistertütchen ein RFID-chip bzw. RFID-tag angeordnet, in welchem der Verabreichungsidentifikationscode einprogrammiert ist.

Mit einem Bestelldatensatz werden vorzugsweise Verblisterungsaufträge für eine bestimmte Zeit von beispielsweise einer Woche oder einem Monat an das Blisterzentrum übertragen. Vorzugsweise werden somit für einen Patient Wochenblister in Form von perforiert aneinander hängenden individuell bedruckten und befüllten Blistertütchen gefertigt. Die fertig gestellten Blister, insbesondere Wochenblister, werden vorzugsweise mit Hilfe eines optischen Kontrollsystems überprüft und dann für den Versand vorbereitet.

In einem weiteren Schritt ST6 werden gefertigten Verabreichungseinheiten, also insbesondere die Blistertütchen verpackt und insbesondere an Apotheken ausgeliefert. Vorzugsweise werden von der Software des Blisterzentrums verschiedene Statusmeldungen an die zentrale Anwendung 14 bzw. den zentralen Applikationsserver 12 versendet, wie z.B. "Auftrag angenommen", "Auftrag wird bearbeitet", "Nachkontrolle", "Versendet". Die fertig gestellten Wochenblister werden über die Logistik an die bestellenden Apotheken versendet und nach einer Eingangskontrolle von der Apotheke an die zu versorgenden Heime ausgeliefert, bzw. an die ambulanten Patienten abgegeben (Schritt ST7).

In einer bevorzugten Ausführungsform werden die Blistertütchen bereits im Blisterzentrum in einen Dispenser, also eine Blisterbox, verpackt, aus der sie nacheinander einzeln in der Reihenfolge ihrer Einnahmezeitpunkte wieder entnommen werden können. In einem Aspekt stellt die Erfindung dazu einen Dispenser bzw. eine Blisterbox bereit, aus der ein Patient oder eine Pflegekraft zum Dosierungszeitpunkt das jeweils oberste Tütchen abreißen und die darin enthaltenen Medikamente entnehmen kann. Vorzugsweise in der Blisterbox ein Barcode-Scanner integriert, der beim Entnehmen bzw. Abreißen des obersten Tütchens automatisch den auf dem Tütchen aufgedruckten Barcodes scannt und registriert. Vorzugsweise umfasst die Blisterbox weiterhin ein Modul zur temporären Speicherung der gescannte bzw. registrierten Daten, z.B. eine handelsübliche SD-Karte. Weiter bevorzugt umfasst die Blisterbox eine Schnittstelle für die Übertragung der gescannten bzw. registrierten bzw. gespeicherten Daten, wie z.B. einen USB-Anschluss oder ein drahtloses Kommunikationsmedium wie IEEE oder ZigBee. Beim Scannen des abgerissenen Tütchens wird vorzugsweise der Barcode gelesen und die Identifikationsnummer zusammen mit einem Zeitstempel gespeichert. Dies geschieht so lange, bis der Medikamentenvorrat in der Box aufgebraucht wurde.

Wenn der Medikamentenvorrat aufgebraucht wurde, wird die Box durch das Blisterzentrum ausgetauscht. Die im Speicher-Modul gespeicherten Daten werden über die Datenübertragungsschnittstelle in ein System für die elektronische Datenverarbeitung gespielt, das direkt oder indirekt mit dem Internet verbunden werden kann. Die Daten gelangen so als Verabreichungskontrolldaten mit Hilfe eines Übertragungsprotokolls in ein weiterverarbeitendes EDV-System für ein Therapie-Adhärenz-Monitoring, wie z.B. im bzw. mit Hilfe der zentralen Anwendung 14. Auf Grundlage der in den Verabreichungskontrolldaten enthaltenen Verabreichungsidentifikation der einzelnen Blistertütchen wird in der Verordnungsdatenbank jeweils derjenige Verabreichungsdatensatz 88 ermittelt, dessen Verabreichungsidentifikation 92 mit der erfassten und übermittelten Verabreichungsidentifikation übereinstimmt und die Verabreichungskontrolldaten, insbesondere die Verabreichungszeitdaten 94, also insbesondere der Zeitpunkt des Auslesens des Strichcodes (z.B. mittels der "intelligenten" Blisterbox), werden dem ermittelten Verabreichungsdatensatz zugeordnet, wie in **Fig. 3C** veranschaulicht.

Die Abgabe der Medikamentenration einer Verabreichungseinheit, insbesondere eines Blistertütchens erfolgt in der beschriebenen Weise vorzugsweise in einem Schritt ST8. In der beschriebenen bevorzugten Ausführungsform stellt die Blisterbox damit eine Verabreichungskontrolleinheit dar. In einer anderen bevorzugten Ausführungsform wird zusätzlich oder alternativ ein optischer Scanner 44 bereitgestellt, der über ein Scan-System 46 an das Netzwerk gekoppelt ist. Vorzugsweise ist der optische Scanner 44 als 1 D- und/oder 2D-Scanner ausgebildet und ausgelegt, die auf den Verabreichungseinheiten, insbesondere den Blistertütchen abgedruckten Strichcodes bzw. Matrixcodes zu erfassen. Dabei ist der optische Scanner 44 vorzugsweise an ein Rechnersystem, wie z.B. einen Desktop-Rechner oder einen mobilen Rechner (Laptop), insbesondere einen PC, als Scan-System 46 angeschlossen, auf dem eine entsprechende Software zur Unterstützung des optischen Scanners 44 und zur Speicherung und/oder Auswertung und/oder Übermittlung des mittels des optischen Scanners 44 erfassten Verabreichungsidentifikationscodes bereitgestellt bzw. installiert ist. Vorzugsweise umfasst das Scan-System 46 eine interne Systemuhr, so dass das Scan-System 46 ausgelegt ist aus dem erfassten Verabreichungsidentifikationscode und dem Zeitpunkt des Erfassens die Verabreichungskontrolldaten zu generieren. Diese können entweder wie bei der beschriebenen Blisterbox zwischengespeichert werden, beispielsweise wenn für das Scan-System 46 temporär keine Netzverbindung zur Verfügung steht, oder sie werden sofort an die zentrale Anwendung 14 übertragen.

Vorzugsweise umfasst das Scan-System 46 eine Ausgabeeinrichtung, insbesondere eine optische und/oder eine akustische Ausgabeeinrichtung. Besonders bevorzugt umfasst das Scan-System ein Display bzw. einen Bildschirm mit einer programmtechnisch eingerichteten grafischen Benutzeroberfläche. Dies ist besonders vorteilhaft, um damit eine sofortige Rückmeldung zur Verifikation des Verabreichungsvorgangs zu ermöglichen. Dazu werden die Verabreichungskontrolldaten vorzugsweise sofort an die zentrale Anwendung 14 übertragen bzw. von der zentralen Anwendung 14 verarbeitet und der zugehörige Verabreichungsdatensatz 88 auf Basis der Verabreichungsidentifikation ermittelt. Anschließend werden auf Grundlage von Patientenidentifikationsdaten, welche demjenigen Verordnungsdatensatz zugeordnet ist bzw. von demjenigen Verordnungsdatensatz umfasst ist, der den ermittelten Verabreichungsdatensatz 88 umfasst, die in der Patientendatenbank zugeordneten Patientenverifikationsdaten ermittelt und zumindest teilweise an die Verabreichungskontrolleinheit übertragen. Vorzugsweise wird also hierbei ein Foto des Patienten und/oder dessen Name zur Verifikation der korrekten Verabreichung an das beispielsweise von Pflegepersonal bediente Scan-System 46 übermittelt und auf dem Display ausgegeben. Damit kann der Benutzer schnell und zuverlässig überprüfen, ob das gescannte Blisterpäckchen tatsächlich für den gewünschten Patienten bestimmt ist. Die graphische Benutzeroberfläche kann dabei eine Eingabe zur Bestätigung durch den Benutzer zulassen. Diese Verifikationsbestätigung wird dann vorzugsweise an die Verordnungsdatenbank bzw. die zentrale Anwendung 14 bzw. den zentralen Applikationsserver 12 übermittelt.

In einer weiteren bevorzugten Ausführungsform bietet das System eine zusätzliche oder alternative Verifikation des Verabreichungsvorgangs. Dazu werden ebenfalls die Verabreichungskontrolldaten vorzugsweise sofort an die zentrale Anwendung 14 übertragen bzw. von der zentralen Anwendung 14 verarbeitet und der zugehörige Verabreichungsdatensatz 88 auf Basis der Verabreichungsidentifikation ermittelt. Anschließend werden auf Grundlage der in dem ermittelten Verabreichungsdatensatz 88 enthaltenen Arzneimittelidentifikationen aus der Arzneimitteldatenbank Arzneimittelverifikationsdaten ermittelt und zumindest teilweise an die Verabreichungskontrolleinheit übertragen. Vorzugsweise wird also hierbei z.B. Arzneimittelbezeichnungen, Arzneimittelfotos oder schematische Zeichnungen bzw. graphische Darstellungen der geometrischen Formen und Besonderheiten des jeweiligen Medikaments (Form, Größe, Farbe, Prägungen, etc.) an das beispielsweise von Pflegepersonal bediente Scan-System 46 übermittelt und auf dem Display ausgegeben. Damit kann der Benutzer schnell und zuverlässig überprüfen, ob das gescannte Blisterpäckchen korrekt befüllt ist. Die graphische Benutzeroberfläche kann dabei eine Eingabe zur Bestätigung durch den Benutzer zulassen. Diese Verifikationsbestätigung wird dann vorzugsweise an die Verordnungsdatenbank bzw. die zentrale Anwendung 14 bzw. den zentralen Applikationsserver 12 übermittelt.

Der optische Scanner 44 bildet damit zusammen mit dem Scan-System 46 eine Verabreichungskontrolleinheit einer bevorzugten Ausführungsform der vorliegenden Erfindung. In einer weiteren bevorzugten Ausführungsform wird in ansonsten vergleichbarer bzw. analoger Weise als Verabreichungskontrolleinheit ein Mobiltelefon 48 mit einer integrierten Kamerafunktion benutzt. Damit ist es möglich, einen Barcode zu fotografieren und durch eine Software die Bildinformationen auszulesen. Hierbei dient vorzugsweise das Display des Mobiltelefons 48 als Ausgabeeinrichtung.

Nach Auslesen des Barcodes baut das Mobiltelefon 48 vorzugsweise eine Verbindung auf und überträgt die aus dem Barcode gelesenen Informationen. Dies kann z.B. durch eine Verbindung mit dem Internet über eine Mobilfunknetz 50 (z.B. via GSM, GPRS oder UMTS, aber auch per SMS) mittels eines Providers 52 erfolgen. Wurde eine Verbindung mit dem Internet aufgebaut, so kann das Mobiltelefon 48 einen automatischen Redirect auf eine für das Mobiltelefon speziell angepasste Seite anzeigen. Die Darstellung dieser Seite ist abhängig von dem durch das Mobiltelefon unterstützten Format, z.B. kann diese Seite eine übliche Internetseite mit Zuschnitt auf das Display eines Mobiltelefons, aber auch ein spezielles Web-Format für Mobiltelefone sein, wie z.B. W@P. Die Anforderung an die Form der Rückmeldung wird dabei vorzugsweise durch das Mobiltelefon mit übertragen.

Durch einen Klick kann der Nutzer nun die mit der ausgelesenen Information im Zusammenhang stehenden Informationen, wie im Zusammenhang mit dem Scan-System 46 bzw. der Blisterbox oben bereits im Detail ausgeführt, in analoger Weise bestätigen und somit auch z.B. eine Medikamenteneinnahme dokumentieren. Wurde eine SMS versendet so erfolgt die Antwort vorzugsweise ebenfalls via SMS. Ambulanten Patienten wird vorzugsweise eine Reminder-Funktion geboten, die mit verschiedenen Geräten, insbesondere mit dem Mobiltelefon 48, nutzbar ist. Der Patient erhält dabei zum vorgesehenen Einnahmezeitpunkt eine Meldung, welche Medikamente er aus welchem Tütchen nun einnehmen muss.

Mit Hilfe der Informationen aus der Datenbank ist es insbesondere in einem Schritt ST9 weiter möglich, mittels eines Adhärenzdatenaufbereitungssystems 54 Auswertungen zur Therapie-Adhärenz des Patienten zu machen. So können beispielsweise auf einer Verlaufskurve die Einnahmezeitpunkte nachvollzogen werden, woraus auf die Therapie-Adhärenz geschlossen werden kann. Diese Informationen können vorzugsweise allen Betreuern dieser Therapie sowie dem Patienten zugänglich gemacht werden, wie beispielsweise dem behandelnden Arzt z.B. durch Einspielen in sein Praxisverwaltungssystem, einer Monitoring-Station durch Einspielen in eine elektronische Patientenakte oder dem Patienten durch Übertragung in seine persönliche elektronische Gesundheitsakte.

Beispielsweise lässt sich in einer bevorzugten Ausführungsform das Abreißen der Tütchen in einer Verlaufskurve mit Datum und Uhrzeit dargestellt und ist somit zu jedem Zeitpunkt nachvollziehbar. Diese Verlaufskurve wird vorzugsweise mit dem Sollzustand aus der Verordnung gemeinsam dargestellt, um Abweichungen und Muster erkenntlich zu machen. Über eine Schnittstelle 56 zu Heimverwaltungssoftware können diese Informationen zu Dokumentationszwecken auch in das verwendete Primärsystem 58 eines Pflegeheims verschickt werden. Vorzugsweise kann auch der behandelnde Arzt jederzeit durch Einsicht auf den Verordnungsplan die Therapie-Adhärenz nachvollziehen und ggf. bei fehlender Therapietreue Kontakt mit dem Patienten aufnehmen. Einige oder mehrere dieser Überwachungs- bzw. Dokumentationsvorgänge werden vorzugsweise in einem Schritt ST10 ausgeführt.

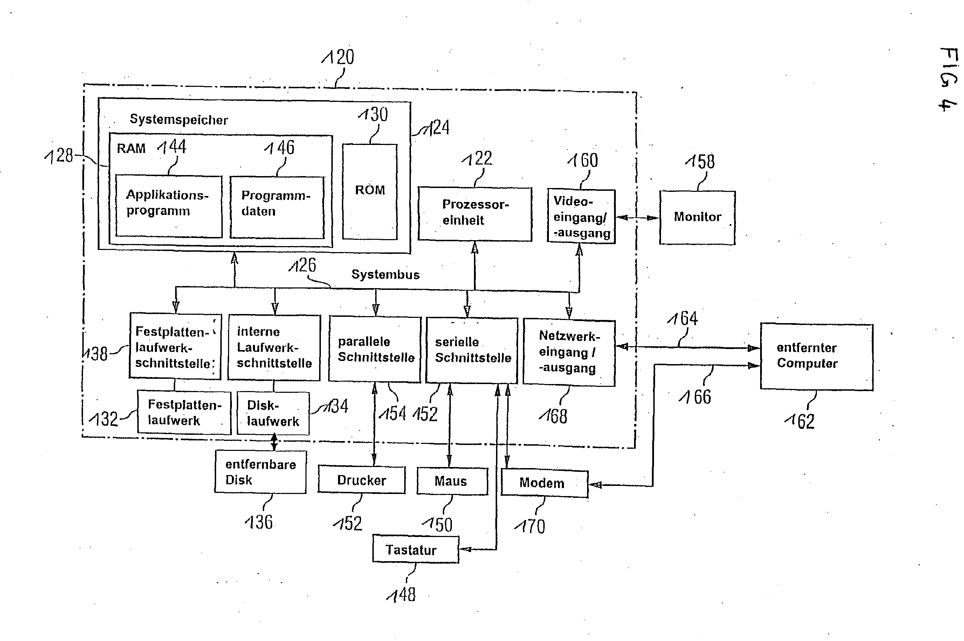

Bezugnehmend auf **Fig. 4** wird ein beispielhaftes System zum Implementieren der Erfindung beschrieben. Ein beispielhaftes System umfaßt eine universelle Rechnereinrichtung in der Form einer herkömmlichen Rechnerumgebung 120 z.B. ein "personal computer" (PC) 120, mit einer Prozessoreinheit 122, einem Systemspeicher 124 und einem Systembus 126, welcher eine Vielzahl von Systemkomponenten, unter anderem den Systemspeicher 124 und die Prozessoreinheit 122 verbindet. Die Prozessoreinheit 122 kann arithmetische, logische und/oder Kontrolloperationen durchführen, indem auf den Systemspeicher 124 zugegriffen wird. Der Systemspeicher 124 kann Informationen und/oder Instruktionen zur Verwendung in Kombination mit der Prozessoreinheit 122 speichern. Der Systemspeicher 124 kann flüchtige und nichtflüchtige Speicher, beispielsweise "random access memory" (RAM) 128 und "Nur-Lesespeicher" (ROM) 130 beinhalten. Ein Grund-Eingabe-Ausgabe-Sytem (BIOS), das die grundlegenden Routinen enthält, welche helfen, Informationen zwischen den Elementen innerhalb des PCs 120, beispielsweise während des Hochfahrens, zu transferieren, kann in dem ROM 130 gespeichert sein. Der Systembus 126 kann eine von vielen Busstrukturen sein, unter anderem ein Speicherbus oder ein Speichercontroller, ein peripherer Bus und ein lokaler Bus, welcher eine bestimmte Busarchitektur aus einer Vielzahl von Busarchitekturen verwendet.

Der PC 120 kann weiterhin ein Festplattenlaufwerk 132 zum Lesen oder Schreiben einer Festplatte (nicht gezeigt) aufweisen und ein externes Disklaufwerk 134 zum Lesen oder Schreiben einer entfernbaren Disk 136 bzw. eines entfernbaren Datenträgers. Die entfernbare Disk kann eine magnetische Disk bzw. eine magnetische Diskette für ein magnetisches Disklaufwerk bzw. Diskettenlaufwerk oder eine optische Diskette wie z.B. eine CD-ROM für ein optisches Disklaufwerk sein. Das Festplattenlaufwerk 132 und das externe Disklaufwerk 134 sind jeweils mit dem Systembus 126 über eine Festplattenlaufwerkschnittstelle 138 und eine externe Disklaufwerkschnittstelle 140 verbunden. Die Laufwerke und die zugeordneten computerlesbaren Medien stellen einen nichtflüchtigen Speicher computerlesbarer Instruktionen, Datenstrukturen, Programm-Modulen und anderer Daten für den PC 120 zur Verfügung. Die Datenstrukturen können die relevanten Daten zum Implementieren eines wie oben beschriebenen Verfahrens aufweisen. Obwohl die beispielshaft beschriebene Umgebung eine Festplatte (nicht gezeigt) und eine externe Disk 142 verwendet, ist für den Fachmann offensichtlich, daß andere Typen computerlesbarer Medien, welche computerzugreifbare Daten speichern können, in der beispielhaften Arbeitsumgebung verwendet werden können, wie z.B. magnetische Kassetten, Flash-Memory Karten, digitale Videodisketten, Random-Access-Speicher, Nur-Lesespeicher, usw..

Eine Vielzahl von Programm-Modulen, insbesondere ein Betriebssystem (nicht gezeigt) ein oder mehrere Applikationsprogramme 144, oder Programm-Module (nicht gezeigt) und Programmdaten 146, können auf der Festplatte, der externen Disk 142, dem ROM 130 oder dem RAM 128 gespeichert werden. Die Applikationsprogramme können zumindest einen Teil der Funktionalität, wie in Fig. 1 gezeigt, umfassen.

Ein Benutzer kann Kommandos und Information, wie oben beschrieben, in den PC 120 anhand von Eingabevorrichtungen, wie z.B. einer Tastatur bzw. eines Keyboards 148 und einer Computermaus 150 eingeben. Andere Eingabevorrichtungen (nicht gezeigt) können ein Mikrofon und/andere Sensoren, einen Joystick, ein Spielpolster bzw. -kissen, einen Scanner oder ähnliches umfassen. Diese oder andere Eingabevorrichtungen können mit der Prozessoreinheit 122 anhand einer seriellen Schnittstelle 152 verbunden sein, welche mit dem System 126 gekoppelt ist, oder können anhand anderer Schnittstellen, wie z.B. einer parallelen Schnittstelle 154, eines Spieleports oder eines universellen seriellen Busses (USB) verbunden sein. Weiterhin kann Information mit einem Drucker 156 gedruckt werden. Der Drucker 156 und andere parallele Eingabe/Ausgabevorrichtungen können mit der Prozessoreinheit 122 durch die parallele Schnittstelle 154 verbunden sein. Ein Monitor 158 oder andere Arten von Anzeigevorrichtung(en) ist/sind mit dem Systembus 126 mittels einer Schnittstelle, wie z.B. eines Videoeingang/-ausgangs 160 verbunden. Zusätzlich zu dem Monitor kann die Rechnerumgebung 120 andere periphere Ausgabevorrichtungen (nicht gezeigt) wie z.B. Lautsprecher oder akustische Ausgänge umfassen.

Die Rechnerumgebung 120 kann mit anderen elektronischen Vorrichtungen z.B. einem Computer, einem Schnurtelefon, einem schnurlosen Telefon, einem persönlichen digitalen Assistenten (PDA), einem Fernseher oder ähnlichem kommunizieren. Um zu kommunizieren, kann die Rechnerumgebung 120 in einer vernetzten Umgebung arbeiten, wobei Verbindungen zu einem oder mehreren elektronischen Vorrichtungen verwendet werden. Fig. 4 stellt die mit einem "remote computer" bzw. entfernten Computer 162 vernetzte Rechnerumgebung dar. Der entfernte Computer 162 kann eine andere Rechnerumgebung, wie z.B. ein Server, ein Router, ein Netzwerk-PC, eine gleichwertige bzw. "peer" Vorrichtung oder andere gewöhnliche Netzwerkknoten sein und kann viele oder alle der hinsichtlich der Rechnerumgebung 120 oben beschriebenen Elemente umfassen. Die logischen Verbindungen, wie sie in Fig. 4 dargestellt sind, umfassen ein "local area network" (LAN) 164 und ein "wide are network" (WAN) 166. Solche Netzwerkumgebungen sind alltäglich in Büros, firmenweiten Computernetzwerken, Intranetzen und dem Internet.

Wenn eine Rechnerumgebung 120 in einer LAN-Netzwerkumgebung verwendet wird, kann die Rechnerumgebung 120 mit dem LAN 164 durch einen Netzwerkeingang/-ausgang 168 verbunden sein. Wenn die Rechnerumgebung 120 in einer WAN-Netzwerkumgebung verwendet wird, kann die Rechnerumgebung 120 ein Modem 170 oder andere Mittel zum Herstellen einer Kommunikation über das WAN 166 umfassen. Das Modem 170, welches intern und extern bezüglich der Rechnerumgebung 120 sein kann, ist mit dem Systembus 126 mittels der seriellen Schnittstelle 152 verbunden. In der Netzwerkumgebung können Programm-Module, welche relativ zu der Rechnerumgebung 120 dargestellt sind, oder Abschnitte davon in einer entfernten Speichereinrichtung gespeichert sein, welche an oder von einem entfernten Computer 162 zugreifbar bzw. systemeigen sind. Weiterhin können andere Daten, welche für das oben beschriebene Verfahren bzw. System relevant sind, auf oder von dem entfernten Computer 162 zugreifbar vorliegen.

### Bezugszeichenliste

- 10: System
- 12: zentraler Applikationsserver
- 14: zentrale Anwendung
- 16: zentraler Datenspeicher
- 18a-c: Anwendersysteme
- 20a-c: Anwendungen
- 22a-c: Schnittstellen
- 24: erstes Verordnersystem
- 30: zweites Verordnersystem
- 32: Browser
- 34: Bestellsystem
- 36: Browser
- 38: Blisterautomat
- 40: Steuerungssoftware
- 42: Schnittstelle
- 44: optischer Scanner
- 46: Scan-System
- 48: Mobiltelefon
- 50: Mobilfunknetz
- 52: Provider
- 54: Adhärenzdatenaufbereitungssystem
- 56: Schnittstelle
- 58: Primärsystem eines Pflegeheims
- 80: Verordnungen bzw. Verordnungsdaten
- 82: Eingabefeld
- 84: "Löschen"-Felder
- 86: Editierfelder
- 88: Verabreichungsdatensätze
- 90: Verordnungszeitdaten
- 92: Verabreichungsidentifikation
- 94: Verabreichungszeitdaten
- 120: Rechnerumgebung
- 122: Prozessoreinheit
- 124: Systemspeicher
- 126: Systembus
- 128: random access memory (RAM)
- 130: Nur-Lesespeicher (ROM)
- 132: Festplattenlaufwerk
- 134: Disklaufwerk
- 136: entfernbare Disk
- 138: Festplattenlaufwerkschnittstelle
- 140: Disklaufwerkschnittstelle
- 142: externe Disk
- 144: Applikationsprogramm
- 146: Programmdaten
- 148: Tastatur
- 150: Computermaus
- 152: serielle Schnittstelle
- 154: parallele Schnittstelle
- 156: Drucker
- 158: Monitor
- 160: Videoeingang/ -ausgang
- 162: entfernter Computer
- 164: "local area network" (LAN)
- 166: "wide are network" (WAN)
- 168: Netzwerkeingang/ -ausgang

## Patentansprüche

1. Computer-implementiertes Verfahren zur Kontrolle bzw. Überwachung einer Verabreichung von Medikamenten an zumindest einen Patienten, umfassend:
- Bereitstellen eines medizinischen Verordnungsdatensatzes für den zumindest einen Patienten in einer Verordnungsdatenbank derart, dass der medizinische Verordnungsdatensatz für eine Vielzahl von Verordnungszeitpunkten jeweils einen Verabreichungsdatensatz festlegt, welcher einer Verabreichungsidentifikation Verordnungszeitdaten und zumindest eine Arzneimittelidentifikation zuordnet;
- Erfassen mittels einer Verabreichungskontrolleinheit eines an einer Verabreichungseinheit angeordneten Verabreichungsidentifikationscodes, in welchem eine Verabreichungsidentifikation kodiert ist;
- Übermitteln von Verabreichungskontrolldaten von der Verabreichungskontrolleinheit an die Verordnungsdatenbank derart, dass die übermittelten Verabreichungskontrolldaten die erfasste Verabreichungsidentifikation und Verabreichungszeitdaten umfassen, welche den Zeitpunkt des Erfassens des Verabreichungsidentifikationscodes festlegen;
- Ermitteln desjenigen Verabreichungsdatensatzes in der Verordnungsdatenbank, dessen in der Verordnungsdatenbank festgelegte Verabreichungsidentifikation mit der erfassten Verabreichungsidentifikation übereinstimmt; und
- Zuordnen der Verabreichungskontrolldaten zu dem ermittelten Verabreichungsdatensatz.

2. Verfahren nach Anspruch 1, wobei der Verabreichungsidentifikationscode als optisch lesbarer Code an der Verabreichungseinheit bereitgestellt wird und das Erfassen des Verabreichungsindentifikationscodes mittels eines optischen Lesegeräts der Verabreichungskontrolleinheit erfolgt.

3. Verfahren nach Anspruch 1 oder 2, wobei in der Verordnungsdatenbank Verordnungsdatensätze für eine Vielzahl von Patienten bereitgestellt werden, denen in der Verordnungsdatenbank jeweils eine Patientenidentifikation zugeordnet ist, und wobei das Verfahren außerdem umfasst:
- Bereitstellen einer Patientendatenbank, welche für eine Vielzahl von Patienten jeweils einer Patientenidentifikation Patientenverifikationsdaten zuweist;
- Ermitteln derjenigen Patientenverifikationsdaten in der Patientendatenbank, deren Patientenidentifikation in der Verordnungsdatenbank demjenigen Verordnungsdatensatz zugeordnet ist, der den ermittelten Verabreichungsdatensatz umfasst;
- Übermitteln der ermittelten Patientenverifikationsdaten an die Verabreichungskontrolleinheit; und
- Ausgeben der übermittelten Patientenverifikationsdaten mittels einer Ausgabeeinrichtung der Verabreichungskontrolleinheit.

4. Verfahren nach einem der vorangegangenen Ansprüche, welches außerdem umfasst:
- Bereitstellen einer Arzneimitteldatenbank, welche für eine Vielzahl von Arzneimitteln jeweils einer Arzneimittelidentifikation Arzneimittelverifikationsdaten zuweist;
- Ermitteln eines Arzneimittelverifikationsdatensatzes derart, dass der Arzneimittelverifikationsdatensatz alle Arzneimittelverifikationsdaten umfasst, die den im ermittelten Verabreichungsdatensatz der erfassten Verabreichungsidentifikation zugeordneten Arzneimittelidentifikationen zugeordnet sind;
- Übermitteln des ermittelten Arzneimittelverifikationsdatensatzes an die Verabreichungskontrolleinheit; und
- Ausgeben der vom übermittelten Arzneimittelverifikationsdatensatz umfassten Arzneimittelverifikationsdaten mittels einer Ausgabeeinrichtung der Verabreichungskontrolleinheit.

5. Verfahren nach einem der Ansprüche 3 oder 4, welches außerdem umfasst:
- Erfassen der Eingabe einer Verifikationsbestätigung an der Verabreichungskontrolleinheit;
- Übermitteln der Verifikationsbestätigung an die Verordnungsdatenbank; und
- Speichern der zugeordneten Verabreichungskontrolldaten zusammen mit dem ermittelten Verabreichungsdatensatz in der Verordnungsdatenbank.

6. Verfahren nach einem der vorangegangenen Ansprüche, wobei das Bereitstellen eines medizinischen Verordnungsdatensatzes für den zumindest einen Patienten ein Festlegen eines Dringlichkeitsparameters für den zumindest einen Patienten und/oder für zumindest einen Verabreichungsdatensatz des zumindest einen Patienten umfasst, und wobei das Verfahren außerdem umfasst:
- Ausgeben einer Warnmeldung, falls nicht innerhalb eines vom Dringlichkeitsparameter abhängigen Toleranzzeitintervalls nach einem in den Verordnungszeitdaten festgelegten Verorndungszeitpunkt dem Verabreichungsdatensatz Verabreichungskontrolldaten zugeordnet wurden.

7. Verfahren nach einem der vorangegangenen Ansprüche, wobei das Erfassen des Verabreichungsidentifikationscodes mittels eines Mobiltelefons mit integrierter Kamera und das Übermitteln der Verabreichungskontrolldaten über ein Mobilfunknetz erfolgt.

8. Verfahren nach einem der vorangegangenen Ansprüche, wobei die Verabreichungskontrolldaten für eine Vielzahl von Verabreichungseinheiten in der Verabreichungskontrolleinheit gespeichert werden.

9. Verfahren nach einem der vorangegangenen Ansprüche, wobei die Verabreichungskontrolleinheit als zumindest ein Teil eines Dispensers für eine Vielzahl von Verabreichungseinheiten derart bereitgestellt wird, dass das Erfassen des an einer Verabreichungseinheit angeordneten Verabreichungsidentifikationscodes beim Ausgeben bzw. Entnehmen der Verabreichungseinheit vom bzw. aus dem Dispenser automatisch erfolgt.

10. Computerimplementiertes Verfahren zur individuellen Verblisterung von Medikamenten für zumindest einen Patienten, umfassend:
- ein Verfahren zur Erstellung und/oder Optimierung eines medizinischen Verordnungsplans;
- Ermitteln zumindest eines Verabreichungsdatensatzes aus dem medizinischen Verordnungsplan;
- automatisches Generieren einer Verabreichungsidentifikation für den zumindest einen Verabreichungsdatensatz;
- Speichern des Verabreichungsdatensatz zusammen mit der Verabreichungsidentifikation in einer zentralen Verordnungsdatenbank;
- Übermitteln des Verabreichungsdatensatzes zusammen mit der jeweiligen Verabreichungsidentifikation und/oder eines daraus generierten Verabreichungsidentifikationcodes an eine automatisch gesteuerte Verblisterungsanlage;
- automatisches Verblistern von Medikamenten gemäß dem Verabreichungsdatensatz, wobei das automatische Verblistern ein Anordnen eines/des aus der Verabreichungsidentifikation generierten Verabreichungsidentifikationcodes an den Blister umfasst.

11. Dispenser zum Bereitstellen von individuell verblisterten Medikamenten, umfassend:
- einen Aufnahmebehälter zum Aufnehmen einer Vielzahl von Verabreichungseinheiten;
- eine Auszugschacht zum Entnehmen einzelner Verabreichungseinheiten;
- eine Leseeinrichtung zum Auslesen von an den Verabreichungseinheiten angeordneten Verabreichungsidentifikationcodes; und
- eine integrierte Zeitmesseinrichtung zum Erfassen von Verabreichungszeitdaten, welche den Zeitpunkt des Erfassens eines Verabreichungsidentifikationcodes zumindest teilweise bzw. relativ festlegen.

12. Dispenser nach Anspruch 11, außerdem umfassend
- eine Ausgabeeinrichtung zum Ausgeben von Verabreichungsverifikationsdaten; und
- eine Eingabeeinrichtung zum Eingeben einer Verifikationsbestätigung.

13. Computersystem zur Kontrolle bzw. Überwachung einer Verabreichung von Medikamenten für zumindest einen Patienten, umfassend:
- eine Verordnungsdatenbank zum Bereitstellen eines medizinischen Verordnungsdatensatzes für den zumindest einen Patienten derart, dass der medizinische Verordnungsdatensatz für eine Vielzahl von Verordnungszeitpunkten jeweils einen Verabreichungsdatensatz festlegt, welcher einer Verabreichungsidentifikation Verordnungszeitdaten und zumindest eine Arzneimittelidentifikation zuordnet;
- Verabreichungskontrolleinheit zum Erfassen eines an einer Verabreichungseinheit angeordneten Verabreichungsindentifikationscodes, in welchem eine Verabreichungsidentifikation kodiert ist, wobei die Verabreichungskontrolleinheit eine Systemuhr umfasst und ausgelegt ist den Zeitpunkt eines Erfassens des Verabreichungsidentifikationscodes als Verabreichungszeitdaten zu erfassen;
- ein Datenübertragungsnetz zum Übermitteln von Verabreichungskontrolldaten von der Verabreichungskontrolleinheit an die Verordnungsdatenbank derart, dass die übermittelten Verabreichungskontrolldaten die erfasste Verabreichungsidentifikation und Verabreichungszeitdaten umfassen;
- eine Analyseeinrichtung zum Ermitteln desjenigen Verabreichungsdatensatz in der Verordnungsdatenbank, dessen in der Verordnungsdatenbank festgelegte Verabreichungsidentifikation mit der erfassten Verabreichungsidentifikation übereinstimmt; und
- eine Zuordnungseinheit zum Zuordnen der Verabreichungskontrolldaten zu dem ermittelten Verabreichungsdatensatz.

14. Computersystem nach Anspruch 13, welches ausgelegt ist, ein Verfahren nach einem der Ansprüche 1 bis 10 auszuführen.

15. Computerprogrammprodukt, welcher Programmcode umfasst, der, wenn er geladen wird auf einem Computer, zur Ausführung eines Verfahrens nach einem der Ansprüche 1 bis 10 geeignet ist.
